# EUROPÄISCHE PATENTANMELDUNG

(11) **EP 4 685 126 A1**
(43) Veröffentlichungstag der Anmeldung: **28.01.2026**
(21) Anmeldenummer: 24190266.7
(22) Anmeldetag: 23.07.2024
(51) Int. Cl.: C07C 2/24, C07C 11/02, C07C 45/50, C07C 47/02

(54) **VERFAHREN ZUR HERSTELLUNG VON C9-ALDEHYDEN BZW. C9-ALKOHOLEN AUS BUTEN-HALTIGEN KOHLENWASSERSTOFFSTRÖMEN**

(71) Anmelder: Evonik Oxeno GmbH & Co. KG, 45772 Marl (DE)
(72) Erfinder: FRANKE, Robert, 45772 Marl (DE); BRÄCHER, Alexander, 55546 Hackenheim (DE); FRIDAG, Dirk, 45721 Haltern am See (DE); MARKOVIC, Ana, 45721 Haltern am See (DE); KÖNIG, Matthias, 45768 Marl (DE); KUCMIERCZYK, Peter, 44628 Herne (DE); SALE, Anna Chiara, 40474 Düsseldorf (DE); BRUNS, Bastian, 44791 Bochum (DE); GEILEN, Frank, 45721 Haltern am See (DE); SIMON, Berno, 45772 Marl (DE)
(74) Vertreter: Evonik Patent Association

(57) **Zusammenfassung**

Die Anmeldung betrifft ein Verfahren zur Herstellung von C9-Alkoholen, vorzugsweise von Isononanol, aus Buten-haltigen Kohlenwasserstoffströmen. Das Verfahren umfasst die Oligomerisierung der Butene, die Hydroformylierung der Oligomerisierungsprodukte zu C9-Aldehyden, vorzugsweise Isononanal, und die Hydrierung der C9-Aldehyde unter Erhalt der C9-Alkohole, vorzugsweise des Isononanols.

## Beschreibung

### Technisches Gebiet

Die vorliegende Erfindung bezieht sich auf das Gebiet der Herstellung von C9-Aldehyden und C9-Alkoholen, einschließlich der Herstellung von Isononanal und Isononanol aus, vorzugsweise, Buten-haltigen Kohlenwasserstoffströmen.

### Hintergrund der Erfindung

Isononanol (INA, für Isononylalkohol) wird zum Beispiel als Rohstoff für die Herstellung von Diisononylphthalat (DINP), einem Phthalatweichmacher, verwendet. INA besteht hauptsächlich aus 3,5,5-Trimethyl-1-hexanol, isomeren Dimethyl-1-heptanolen und isomeren Methyl-1-octanolen (z. B. 7-Methyloctan-1-ol). Die genaue Zusammensetzung schwankt je nach den verwendeten Ausgangsstoffen, dem angewendeten Herstellverfahren, den Reaktionsbedingungen und der Methode der Aufreinigung.

INA wird im industriellen Maßstab durch Hydrierung von Isononanal (INAL, für Isononylaldehyd) hergestellt. INAL wiederum ist durch Hydroformylierung C8-reicher Olefingemische erhältlich. Die zur Herstellung von INA benötigten C8-reichen Olefingemische werden dabei durch Oligomerisierung von Olefinen in flüssiger Phase gewonnen, wobei insbesondere Propen und n-Buten als Ausgangsstoffe dienen.

Ein im Stand der Technik bekanntes Verfahren zur Herstellung von Olefinen, zum Beispiel von C6-, C7- oder C8-reichen Olefingemischen, ist das Dimersol-Verfahren. Unter dem Dimersol-Verfahren wird allgemein eine homogen katalysierte Oligomerisierung von Olefinen in flüssiger Phase bei Temperaturen von 40 bis 60 °C und Drücken bis ca. 1,5 MPa zur Aufrechterhaltung der flüssigen Phase verstanden. Beim sogenannten Dimersol-X-Verfahren wird zur Gewinnung von Octenen aus n-Butenen ein homogener Nickelkatalysator in Gegenwart eines Aluminiumalkyls als Cokatalysator eingesetzt.

Im Dimersol-X-Verfahren werden die Komponenten in den Zulauf dosiert und nach einmaligem Durchlauf durch die Anlage in einer alkalisch-wässrigen Wäsche abgetrennt, woran sich eine destillative Aufarbeitung des Austrags anschließt. Eine Katalysatorrückführung ist üblicherweise nicht vorgesehen und ohne weiteres auch nicht möglich. Es fällt kontinuierlich eine stark alkalische nickel- und aluminiumhaltige Waschlauge an, welche separat aufgearbeitet bzw. entsorgt werden muss.

Im Stand der Technik ist ein zum ursprünglichen Dimersol-X-Verfahren verbessertes Verfahren beschrieben, welches unter dem Namen Difasol-Verfahren bekannt ist. Dieses Verfahren ist durch einen heterogenisierten Zweiphasenprozess mit optimierter Katalysatorabtrennung gekennzeichnet, bei dem der Nickel-Katalysator in einer ionischen Flüssigkeit im Prozess verbleibt.

Für das Teilverfahren zur Hydroformylierung C8-reicher Olefingemische kann zum Beispiel das in der WO 2020/240194 beschriebene LP-Oxo-Verfahren verwendet werden. In diesem Verfahren wird ein C8-Olefin in der Flüssigphase mit einem Ligand-Rhodium-Katalysator hydroformyliert und somit zu einem C9-Aldehyd umgesetzt. Der folgend genannte Stand der Technik gibt einen Überblick über Aspekte der Hydroformylierung wie sie als Teilverfahren im hier beschriebenen Verbundverfahren zur Anwendung kommt.

In der WO 2020/240194 A1 wird ein Verfahren zur Hydroformylierung von Olefinen zu Aldehyden beschrieben. Der Schwerpunkt in diesem Dokument liegt auf der Abtrennung von Schwersiedern und INAL, indem der flüssige Abstrom der Hydroformylierungsreaktion im Gleichstrom mit Kohlenmonoxid durch einen Fallfilmverdampfer geleitet wird. Der Verdampfer wird bei 1,5 bar und 120 °C betrieben. Der Schwersieder, der den Rh-Katalysator enthält, wird in die Reaktionszone zurückgeführt, und das INAL im Dampfstrom wird kondensiert und im Entspannungsgefäß von CO getrennt.

In der WO 2021/091687 A1 wird ein Verfahren zur Rückgewinnung von Rhodium aus einem Hydroformylierungsverfahren beschrieben. Das Verfahren, welches auf experimentellen Ergebnissen beruht, kann in vier Abschnitte unterteilt werden: Behandlung eines Spülstroms eines Hydroformylierungsprozesses, der den Ligand-Rh-Komplex-Katalysator enthält, mit einem Oxidationsmittel und einer halogenidfreien Säure. Der Ligand wird oxidiert und Rh aus der organischen Phase (Schwersieder) in die wässrige Phase überführt. Rückgewinnung der wässrigen Phase. Kontaktierung der wässrigen Phase mit nicht umgesetzten Olefinen aus einem Hydroformylierungsprozess und Ligand unter Synthesegasatmosphäre. Dabei wird der Ligand-Rh-Komplex in der organischen Phase konzentriert. Waschen der organischen Phase mit einem wasserlöslichen Amin.

in der WO 2022/038350 A1 wird ein Verfahren zur Hydroformylierung von C8-Olefingemischen mit Entfernung des gelösten Wasserstoffs beschrieben. Es wurde festgestellt, dass der Ligand-Rh-Komplex unter CO-Atmosphäre stabiler ist und dass Rh-Verluste durch die Entfernung von H₂ reduziert werden kann. In dem Dokument wird ein Verfahren zur Entfernung von Wasserstoff aus dem flüssigen Abstrom des Hydroformylierungsreaktors beansprucht. Der flüssige Abstrom des Reaktors wird im Gegenstrom mit Kohlenmonoxid in eine Stripperkolonne geleitet. Die Kolonne arbeitet bei 14,5 bar und 90 °C. Die Wasserstoffkonzentration im flüssigen Reaktorabstrom wird von 0,5 Mol-% auf 0,02 Mol-% reduziert.

In der WO 2020/112373 wird ein Hydroformylierungsverfahren beschrieben. Einer der wichtigsten Abschnitte im Hydroformylierungsprozess von C8-Olefinen mit Ligand-Rh-Katalysatoren ist die Abtrennung von Schwersiedern aus dem Reaktorabstrom. Der Rh-Komplex ist sehr empfindlich gegenüber hohen Temperaturen und Vakuumbedingungen. In diesem Dokument wird behauptet, dass bei der Abtrennung von Schwersiedern in einem Verdampfer mit Kohlenmonoxid die Zurechenbarkeit von Rh verbessert werden kann, indem die Konzentration von C8-Olefinen und/oder gemischtem C9 > 1,2 Gew.-% im Endstrom des Verdampfers gehalten wird.

Aufgabe der vorliegenden Erfindung ist es, im Rahmen eines Verbundverfahrens zur Herstellung von Isononanol das Teilverfahren der Hydroformylierung C8-reicher Olefingemische zu optimieren.

### Zusammenfassung der Erfindung

In einem ersten Aspekt ist die Erfindung ein Verfahren nach Anspruch 1 zur Herstellung von C9-Aldehyden, optional zur Herstellung von C9-Alkoholen. Das Verfahren umfasst die Schritte: A) Bereitstellen eines n-Buten-haltigen Kohlenwasserstoffstroms; B) Oligomerisieren des Kohlenwasserstoffstroms mithilfe eines homogenen Katalysatorsystems, welches Nickel und ein Aluminiumalkyl umfasst; unter Erhalt eines Oligomerisats, welches C8-Olefine enthält; C) Abtrennen des Katalysatorsystems aus dem Oligomerisat und destillative Aufarbeitung des vom Katalysatorsystem befreiten Oligomerisats, wodurch ein Oligomerisierungsprodukt erhalten wird, welches mindestens 70 Gew.-% C8-Olefine enthält; D) Hydroformylieren einer Zusammensetzung enthaltend C8-Olefine oder Hydroformylieren des Oligomerisierungsprodukts in Anwesenheit von Synthesegas mithilfe eines Katalysatorsystem, welches Cobalt oder Rhodium und einen Liganden umfasst, unter Erhalt einer Hydroformylierungsmischung, welche zumindest C9-Aldehyde enthält; und E) optional, Hydrieren der C9-Aldehyde unter Erhalt von Isononanol.

In einem zweiten Aspekt ist die Erfindung eine Anlage nach Anspruch 10 geeignet zur Durchführung des Verfahrens gemäß Anspruch 1. Die Anlage umfasst die Elemente: A-B) eine Teilanlage zum Oligomerisieren eines n-Buten-haltigen Kohlenwasserstoffstroms; C) eine Teilanlage zum Aufarbeiten des erhaltenen Oligomerisats; D₁₋₁₀) eine Teilanlage zum Hydroformylieren; und E) optional, eine Teilanlage zum Hydrieren von C9-Aldehyden. Die baulichen Merkmale der Teilanlagen A) bis E) sind im Anspruch 10 definiert.

### Beschreibung der Zeichnungen

Abbildung 1 zeigt ein vereinfachtes Schema des Ablaufs des Teilverfahrens der Hydroformylierung mit den Abschnitten 100, 200, 300, 400, 500 und 600 des erfindungsgemäßen Verfahrens mit wesentlichen sowie optionalen Merkmalen.

Abbildung 2 zeigt ein detaillierteres Schema des Ablaufs des Teilverfahrens der Hydroformylierung mit den Abschnitten 100, 200, 300, 400, 500 und 600 des erfindungsgemäßen Verfahrens mit wesentlichen sowie optionalen Merkmalen.

Abbildung 3 zeigt ein detaillierteres Schema des Ablaufs des Abschnitts 600 des Teilverfahrens der Hydroformylierung des erfindungsgemäßen Verfahrens.

### Beschreibung der Erfindung

Die oben beschriebene Aufgabe der Erfindung wird gelöst durch ein Verfahren zur Herstellung von C9-Aldehyden, wobei das Verfahren nachfolgende Schritte umfasst:
A) Bereitstellen eines Kohlenwasserstoffstroms, der n-Butene enthält;
B) Oligomerisieren des Kohlenwasserstoffstroms mithilfe eines homogenen Katalysatorsystems, welches Nickelverbindung und ein Aluminiumverbindung umfasst, unter Erhalt eines Oligomerisats, welches C8-Olefine enthält; und
C) Abtrennen des Katalysatorsystems aus dem Oligomerisat und destillative Aufarbeitung des vom Katalysatorsystem befreiten Oligomerisats, wodurch ein Oligomerisierungsprodukt als Zusammensetzung (010) erhalten wird, welches mindestens 70 Gew.-% C8-Olefine enthält;
D₁) Zuführen einer Zusammensetzung (010) aus Schritt C) in einen Reaktor (100) und Zuführen einer Mischung von Wasserstoff H₂ und Kohlenmonoxid CO, also Synthesegas (020b), in denselben Reaktor (100) unter Erhalt einer Reaktionsmischung;
D₂) Hydroformylieren der Reaktionsmischung in dem Reaktor (100) mithilfe eines Katalysatorsystems, welches Cobalt oder Rhodium und einen Liganden umfasst, unter Erhalt einer Hydroformylierungsmischung (100a), welche zumindest C9-Aldehyde, vorzugsweise Isononanal, enthält;
D₃) Zuführen der Hydroformylierungsmischung (100a) in ein Entspannungsgefäß (101), dortiges Abtrennen einer Gasphase (101a) und einer Flüssigphase (101b) aus der Hydroformylierungsmischung (100a) und Überführen der Flüssigphase (101b) in eine Stripkolonne (200);
D₄) Zuführen von Kohlenmonoxid CO (400b) in die Stripkolonne (200), dortiges Abtrennen einer Gasphase (200a) und einer Flüssigphase (200b) aus der Flüssigphase (101b) und Überführen der Flüssigphase (200b) in einen Verdampfer (300);
D₅) Zuführen eines CO-haltigen Gases (302a1) in den Verdampfer (300), dortiges Abtrennen einer Gasphase (300a) und einer Flüssigphase (300b) und Überführen der Gasphase (300a) in einen Kondensator (301) und Überführen des Kondensats (300c) in ein Entspannungsgefäß (302);
D₆) in dem Entspannungsgefäß (302) Abtrennen einer Gasphase (302a) und einer C9-Aldehyd-haltigen Flüssigphase (302b) aus der Flüssigphase (300c);
D₇) Zuführen der Gasphase (101a) aus Schritt D₃ in einen Kondensator (102), in dem Kondensator (102) Abtrennen eines Kondensats (102b) und Rückführen des Kondensats (102b) in das Entspannungsgefäß (101), und in dem Kondensator (102) Abtrennen eines Recyclingsynthesegases (102a) und Rückführen des Recyclingsynthesegases (102a) in den Reaktor (100);
D₈) Zuführen der Gasphase (200a) aus Schritt D₄ in einen Kondensator (201), in dem Kondensator (201) Abtrennen eines Kondensats (201b) und Rückführen des Kondensats (201b) in die Stripkolonne (200), und in dem Kondensator (201) Abtrennen eines Recyclingsynthesegases (201a) und Rückführen des Recyclingsynthesegases (201a) in den Reaktor (100);
D₉) Anreichern eines Teils der Gasphase (302a) aus Schritt D₆ mit Kohlenmonoxid CO (400b) und Rückführen des so erhaltenen CO-haltigen Gases (302a1) in den Verdampfer (300);
D₁₀) Anreichern eines verbleibenden Teils (302a2) der Gasphase (302a) mit Wasserstoff H₂ (400a) unter Bildung eines Recyclingsynthesgas (400a1) und Rückführen desselben in den Reaktor (100);
dadurch gekennzeichnet, dass von dem Recyclingsynthesgas (102a) eine solche Gasmenge als Purgestrom (102aP1), von dem Recyclingsynthesgas (201a) eine solche Gasmenge als Purgestrom (201 aP2) und/oder von dem CO-haltigen Gas (302a2) eine solche Gasmenge als Purgestrom (302aP3) entnommen und verworfen wird, dass die Summe der Recyclinggasströme (102a), (201a) und (400a1) ein Molverhältnis H₂ zu CO im Bereich von 0,9 bis 1,1 aufweist, oder dass die Recyclinggasströme (102a), (201a) und (400a1) mit frischem Synthesegas (020a) gemischt werden und das dadurch gebildete Synthesegas (020b) ein Molverhältnis H₂ zu CO im Bereich von 0,9 bis 1,1 aufweist.

Das erfindungsgemäße Verfahren zur Herstellung von C9-Aldehyden umfasst die Herstellung der benötigten Zusammensetzung enthaltend mindestens 70 Gew.-% C8-Olefine. Die Herstellung umfasst die den Schritten D₁) bis D₁₀) vorangestellten nachfolgenden Schritte:
A) Bereitstellen eines Kohlenwasserstoffstroms, der n-Butene enthält;
B) Oligomerisieren des Kohlenwasserstoffstroms mithilfe eines homogenen Katalysatorsystems, welches Nickelverbindung und ein Aluminiumverbindung umfasst, unter Erhalt eines Oligomerisats, welches C8-Olefine enthält; und
C) Abtrennen des Katalysatorsystems aus dem Oligomerisat und destillative Aufarbeitung des vom Katalysatorsystem befreiten Oligomerisats, wodurch ein Oligomerisierungsprodukt als Zusammensetzung (010) erhalten wird, welches mindestens 70 Gew.-% C8-Olefine enthält.

In Schritt A des erfindungsgemäßen Verfahrens enthält der bereitgestellte Kohlenwasserstoffstrom vorzugsweise einen Gehalt von 30 bis 100 Gew.-% n-Buten. Typische Bereiche hängen davon ab, in welchen Produktionsverbund dieses Verfahren integriert wird. Wenn eine Butan-Buten-Trennung eingesetzt wird, können Ströme mit über 98 Gew.-% n-Buten zur Verfügung stehen. Wird jedoch zum Beispiel ein C4-Raffinat eingesetzt, kann ein Gehalt von 30 bis 85 Gew.-% n-Buten im Strom bereitgestellt werden. Sollen weitere Nebenströme verwertet werden, kann der Gehalt an Olefinen auch niedriger liegen. Unterhalb eines Gehalts von 20 Gew.-% n-Buten ist eine wirtschaftliche Oligomerisierung kaum noch möglich.

Das bei der Oligomerisierung in Schritt B) eingesetzte homogene Katalysatorsystem enthält eine Nickelverbindung und ein Aluminiumalkyl. Entsprechende homogene Katalysatorsysteme sind dem Fachmann bekannt, beispielsweise aus der US 2013/0158321 A1.

Die Nickelverbindung für das Katalysatorsystem in Schritt B) kann ein Nickelcarboxylat sein, bei dem Nickel als Nickel (II) vorliegt und zwei Carbonsäuresalzgruppen aufweist. Geeignete Säuresalze sind Octoat, 2-Ethylhexanoat, Decanoat, Stearat, Oleat, Salicylat und Hydroxydecanoat. Ein besonders bevorzugtes Nickelcarbonxylat ist Nickel(II)-ethylhexanoat. Die Nickelverbindung kann nach der vorliegenden Erfindung auch ein Nickel-Komplex sein. Ein Beispiel dafür ist Nickel(II)-acetylacetonat. Andere geeignete Komplexe sind Komplexe von Nickelhalogeniden, Nickelsulfaten, Nickeloctoat oder Nickelalkoholaten. Es können aber auch Nickel-Komplexe mit einer Wertigkeit von weniger als 2 eingesetzt werden, beispielsweise Nickelbiscyclooctadien, Methallylnickelchlorid, Bisallylnickel, Allylnickelchlorid und Allylnickelbromid.

Geeignete Nickelverbindungen sind vorzugsweise Nickel(II)-chlorid, Nickel(II)(dimethoxyethan)-chlorid, Nickel(II)-bromid, Nickel(II)(dimethoxyethan)-bromid, Nickel(II)-fluorid, Nickel(II)-iodid, Nickel(II)-sulfat, Nickel(II)-carbonat, Nickel(II)-dimethylglyoxim, Nickel(II)-hydroxid, Nickel(II)-hydroxyacetat, Nickel(II)-oxalat, Nickel(II)-carboxylate wie zum Beispiel Nickel-2-ethylhexanoat, Nickel(II)-phenate, Nickel(II)-naphthenate, Nickel(II)-acetat, Nickel(II)-trifluoracetat, Nickel(II)-triflat, Nickel(II)-acetylacetonat, Nickel(II)-hexafluoroacetylacetonat, n-Allylnickel(II)-chlorid, n-Allylnickel(II)-bromid, Methallylnickel(II)-chlorid-Dimer, η3-Allylnickel(II)-hexafluorophosphat, η3-Methallylnickel(II)-hexafluorophosphat, Nickel(II)-1,5-Cyclooctadienyl oder Mischungen davon. Diese Nickelverbin in ihrer hydratisierten oder nichthydratisierten Form,

Die Aluminiumverbindung für das Katalysatorsystem in Schritt B) kann eine Alkylaluminiumverbindung, eine Mischung von Alkylaluminiumverbindungen, eine Halogenoalkylaluminiumverbindung oder eine Mischung von Halogenoalkylaluminiumverbindungen sein. Geeignete Aluminiumverindungen sind Metyhlaluminiumdichlorid (MeAlCl₂), Ethylaluminiumdichlorid (EtAlCl₂), Diethylaluminiumchlorid (Et₂AlCl), Diisobutylaluminiumchlorid (iBu₂AlCl) und Isobutylaluminiumdichlorid (iBuAlCl₂). Eine besonders bevorzugte Aluminiumverbindung ist Ethylaluminiumdichlorid (EtAlCl₂) oder Mischungen davon. Möglich ist auch der Einsatz von Halogenoaluminaten oder Organohalogenoaluminaten, vorzugsweise als kationischer Teil einer ionischen Flüssigkeit.

Das Katalysatorsystem für die Oligomerisierung in Schritt B) kann je nach spezieller Ausführungsform zusätzliche Komponenten enthalten.

Das Katalysatorsysten kann beispielsweise eine Brönsted-Säure enthalten. Geeignete Brönsted-Säuren sind Carbon- oder Sulfonsäuren und deren Derivate. Eine bevorzugte Klasse sind Halogenocarbonsäuren. Beispiele hierfür sind Trifluoressigsäure, Difluoressigsäure, Flouressigsäure, Trichloressigsäure, Dichloressigsäure und Chloroessigsäure. Es ist aber auch möglich, Arilsulfonsäuren, Alkylsulfonsäuren, Fluoroalkylsulfonsäuren, Pikrinsäure oder Nitroessigsäure zu verwenden. Besonders bevorzugt wird Trifluoressigsäure als Brönsted-Säure eingesetzt.

Das Katalysatorsystem für Schritt B) kann auch eine Lewis-Säure enthalten, sofern keine Brönsted-Säure anwesend ist. Geeignete Lewis-Säuren sind Diethylether, Methyl-tert-butylether, Tetrahydrofuran, 1,4-Dioxane, Isoxazol, Pyridin, Pyrazin und Pyrimidin.

Es ist auch möglich, dass das Katalysatorsystem in Schritt B) ein organisches Polyol wie Ethylenglykol umfasst. Weiterhin kann eine Verbesserung der Selektivität durch die Anwesenheit von Organophosphorliganden erreicht werden. Beispiele für geeignete Liganden sind Phosphinliganden, die drei identische Kohlenwasserstoffgruppen umfassen. Beispiele für Kohlenwasserstoffgruppen sind Phenyl, o-Tolyl, m-Tolyl, p-Tolyl, Mesityl, 3,5-Dimethylphenyl, 4-n-Butylphenyl, 4-Methoxyphenyl, 2-Methoxyphenyl, 3-Methoxyphenyl, 2-Isopropoxyphenyl, 4-Methoxy-3,5-dimethylphenyl, 3,5-Di-tert-butyl-4-methoxyphenyl, 4-Chlorophenyl, 3,5-Di(trifluoromethyl)phenyl, Benzyl, Naphthyl, Bisnaphthyl, Pyridyl, Bisphenyl, Furanyl, Thiophenyl, Ethyl, Propyl, Isopropyl, n-Butyl, tert-Butyl, Cyclopentyl, Cyclohexyl, und Adamantyl. Bevorzugte Phosphinliganden sind Tri-n-Butylphosphin, Tricyclohexylphosphin, Triisopropylphosphin oder Triphenylphosphin. In einer bevorzugten Ausführungsform liegt das molare Verhältnis des Phosphinliganden zur Nickelverbindung im Bereich von 5 bis 25, vorzugsweise im Bereich von 5 bis 20, noch bevorzugter im Bereich von 5 bis 15.

Sofern das Katalysatorsystem einen Nickel-Komplex enthält, kann die Oligomerisierung in Schritt B) zweiphasig unter Einsatz einer ionischen Flüssigkeit durchgeführt werden. Die ionische Flüssigkeit umfasst dabei vorzugsweise mindestens ein Salz mit der Formel Q+A-, wobei Q+ ein quartäres Ammonium-Kation, ein Phosphonium-Kation, eine Mischung aus Ammonium- und Phosphonium-Kation oder ein Lithium-Kation ist und A- ein koordinierendes oder nicht-koordinierendes Anion ist, das aus der Gruppe aus Halogenoaluminaten, Organohalogenoaluminaten, Organogallaten, Organohalogenogallaten oder einer Mischung aus mindestens zwei dieser Verbindungen ausgewählt wird.

Ohne den Einsatz einer ionischen Flüssigkeit liegt eine einphasige flüssige Mischung des Katalysatorsystem und bei der Oligomerisierung in Schritt b) vor. Die Komponenten des Katalysatorsystem können dabei in einem Lösungsmittel bei kontrollierter Temperatur und für eine bestimmte Zeit vermischt werden. Als Lösungsmittel eignen sich Alkane, aromatische Kohlenwasserstoffe, halogenierte Kohlenwasserstoffe oder die bei der Oligomerisierung in Schritt B) produzierten Olefine, hier also C8-Olefine. Das Vermischen unter Rühren erfolgt vorzusgweise unter einer inerten Atmosphäre aus Stickstoff oder Argon. Die Temperatur beim Vermischen liegt vorzugsweise im Bereich von 0° C. bis 80° C., vorzugsweise im Bereich von 10° C. bis 60° C.

Die Oligomerisierung in Schritt B) wird vorzugsweise bei einer Temperatur von -20° C. bis 80° C, vorzugsweise bei einer Temperatur von 20 bis 60 °C. unter Druckbedingungen durchgeführt, damit die Reaktionsmischung in der flüssigen Phase oder in einer kondensierten Phase gehalten wird.

Die Oligomerisierung in Schritt B) kann weiterhin in einem oder mehreren Reaktoren durchgeführt werden. Geeignete Reaktoren sind dem Fachmann bekannt. Ein Beispiel für einen geeigneten Reaktor ist ein gerührter Reaktor (CSTR / Continous stirrend tank reactor). In einer bevorzugten Ausführungsform wird die Oligomerisierung in einer Reaktionszone durchgeführt, die mehrere identische oder unterschiedliche Reaktoren enthält. Die Reaktoren können parallel oder in Reihe geschaltet vorliegen. Bevorzugt liegt eine Kaskade aus mehreren hintereinander geschalteten Reaktoren vor. Jeder Reaktor kann dabei über eine separate Dosierung des Katalysatorsystem verfügen.

Bei der Oligomerisierung fällt ein Oligomerisat an, welches die C8-Butene enthält. Zusätzlich kann im Oligomerisat auch das homogen gelöste Katalysatorsystem vorliegen.

Aus dem Oligomerisat wird anschließend das Katalysatorsystem abgetrennt. Dabei wird das Katalysatorsystem zunächst zerstört. Entsprechende Verfahren sind dem Fachmann bekannt. Das kann zum Beispiel durch eine alkalische Wäsche mit einer alkalischen Waschlauge geschehen, bei dem das Nickel in die wässrige Phase übertritt. Anschließend kann eine wässrige Wäsche durchgeführt werden, um eventuell mitgeschleppte alkalische Waschlauge abzutrennen. Das restliche Oligomerisat verbleibt als organische Phase.

Das vom Katalysatorsystem befreiten Oligomerisat wird anschließend einer destillativen Aufarbeitung zugeführt, wodurch ein Oligomerisierungsprodukt als Zusammensetzung (010) erhalten wird, welches mindestens 70 Gew.-% C8-Olefine enthält.

Destillationsverfahren sind dem Fachmann bekannt. Die Bedingungen der Destillation, also beispielsweise Temperatur und Druck, sind üblicherweise durch den Aufbau (Höhe Kolonne, Anzahl der Böden, Art der Böden bzw. Packung, Abstände usw.) festgelegt. Während des Betriebs können die Trenneigenschaften der Destillation noch über die Temperaturverteilung und/oder die Wärmezufuhr in der Kolonne und den Rücklauf im Destillat gesteuert werden. Ebenso kann die Trenneigenschaft durch die Veränderung des Drucks in einem gewissen Rahmen eingestellt werden. Die genauen Einstellungen lassen sich daher nicht übergeordnet und unabhängig von dem Aufbau der Destillationskolonne definieren. Das ist dem Fachmann bekannt. In einer bevorzugten Ausführungsform liegt der Druck bei der Destillation in Schritt C) im Bereich von 1 bis 6 bar absolut, besonders bevorzugt im Bereich von 2 bis 5 bar absolut. Die Temperatur am Kopf der Destillationskolonne liegt weiterhin bevorzugt im Bereich von 15 bis 60 °C, besonders bevorzugt im Bereich von 25 bis 50 °C.

Nach der Herstellung der C8-Olefine bzw. der Zusammensetzung (010) erfolgen die Schritte D₁) bis D₁₀):
D₁) Zuführen der Zusammensetzung (010) aus Schritt C) in einen Reaktor (100) und Zuführen einer Mischung von Wasserstoff H₂ und Kohlenmonoxid CO, also Synthesegas (020b), in denselben Reaktor (100) unter Erhalt einer Reaktionsmischung;
D₂) Hydroformylieren der Reaktionsmischung in dem Reaktor (100) mithilfe eines Katalysatorsystems, welches Cobalt oder Rhodium und einen Liganden umfasst, unter Erhalt einer Hydroformylierungsmischung (100a), welche zumindest C9-Aldehyde, vorzugsweise Isononanal, enthält;
D₃) Zuführen der Hydroformylierungsmischung (100a) in ein Entspannungsgefäß (101), dortiges Abtrennen einer Gasphase (101a) und einer Flüssigphase (101b) aus der Hydroformylierungsmischung (100a) und Überführen der Flüssigphase (101b) in eine Stripkolonne (200);
D₄) Zuführen von Kohlenmonoxid CO (400b) in die Stripkolonne (200), dortiges Abtrennen einer Gasphase (200a) und einer Flüssigphase (200b) aus der Flüssigphase (101b) und Überführen der Flüssigphase (200b) in einen Verdampfer (300);
D₅) Zuführen eines CO-haltigen Gases (302a1) in den Verdampfer (300), dortiges Abtrennen einer Gasphase (300a) und einer Flüssigphase (300b) und Überführen der Gasphase (300a) in einen Kondensator (301) und Überführen des Kondensats (300c) in ein Entspannungsgefäß (302);
D₆) in dem Entspannungsgefäß (302) Abtrennen einer Gasphase (302a) und einer C9-Aldehyd-haltigen Flüssigphase (302b) aus der Flüssigphase (300c);
D₇) Zuführen der Gasphase (101a) aus Schritt D₃ in einen Kondensator (102), in dem Kondensator (102) Abtrennen eines Kondensats (102b) und Rückführen des Kondensats (102b) in das Entspannungsgefäß (101), und in dem Kondensator (102) Abtrennen eines Recyclingsynthesegases (102a) und Rückführen des Recyclingsynthesegases (102a) in den Reaktor (100);
D₈) Zuführen der Gasphase (200a) aus Schritt D₄ in einen Kondensator (201), in dem Kondensator (201) Abtrennen eines Kondensats (201b) und Rückführen des Kondensats (201b) in die Stripkolonne (200), und in dem Kondensator (201) Abtrennen eines Recyclingsynthesegases (201a) und Rückführen des Recyclingsynthesegases (201a) in den Reaktor (100);
D₉) Anreichern eines Teils der Gasphase (302a) aus Schritt D₆ mit Kohlenmonoxid CO (400b) und Rückführen des so erhaltenen CO-haltigen Gases (302a1) in den Verdampfer (300);
D₁₀) Anreichern eines verbleibenden Teils (302a2) der Gasphase (302a) mit Wasserstoff H₂ (400a) unter Bildung eines Recyclingsynthesgas (400a1) und Rückführen desselben in den Reaktor (100);

Die in Schritt D₆) abgetrennte C9-Aldehyd-haltige Flüssigphase (302b) enthält das gewünschte Herstellungsprodukt des erfindungsgemäßen Verfahrens. Zur Isolierung des Herstellungsprodukts kann die Flüssigphase (302b) in eine Destillationskolonne (500) zur Abtrennung der C9-Aldehyde (500a), vorzugsweise zur Abtrennung von Isononanal, überführt werden.

Das erfindungsgemäße Verfahren kann zu einem Verfahren zur Herstellung von C9-Alkoholen erweitert werden. In einem solchen Verfahren schließt sich an den Schritte D₁) bis D₁₀) ein Schritt E) an, in welchem die erhaltenen C9-Aldehyde (500a), vorzugsweise Isononanal, unter Erhalt von C9-Alkoholen, vorzugsweise von Isononanol, hydriert werden. Die Hydrierung ist ein dem Fachmann bekanntes Verfahren und wird hier nicht näher beschrieben.

Die Hydroformylierung der Reaktionsmischung in Schritt D₂ kann auf verschiedene Weise und bei unterschiedlichen Reaktionsbedingungen durchgeführt werden. So kann die Hydroformylierung bei einer Temperatur im Bereich von 90 bis 150 °C, vorzugsweise 110 bis 130 °C, und bei einem Druck im Bereich von 10 bis 40 bar, vorzugsweise 15 bis 25 bar, durchgeführt werden.

Als Katalysatorsystem kann Rhodium in einer Konzentration von 5 ppm bis 500 ppm, vorzugsweise 100 ppm bis 300 ppm jeweils bezogen auf die gesamte Rektionsmischung in der Hydroformylierung, mit Alkanox 240 (= Tris(2,4-di-tert-butylphenyl)phosphit, CAS-Nr.: 31570-04-4) als Liganden P in einer Konzentration von 1 mol P/mol Rh bis 50 mol P/mol Rh verwendet werden.

Die in Schritt D₂ erhaltene Hydroformylierungsmischung enthält typischerweise 60 bis 90 Gew.-%, vorzugsweise 70 bis 80 Gew.-% C9-Aldehyde, 10 bis 30 Gew.-% nicht reagierte C8-Olefine, 0,01 bis 5 Gew.-% Nebenprodukte (zum Beispiel Paraffine, Alkohole und andere Verbindungen), 0 bis 5 Gew.-% Schwersieder, sowie 0 bis 5 Gew.-% Synthesegas. Es versteht sich, dass die Summe aller Komponenten zusammen 100 Gew.-% ergeben.

In Schritt D₄ kann reines Kohlenmonoxid (>99 Gew.-% CO) zugeführt werden, es können aber Gasmischungen eingesetzt werden, die eine hohen Anteil, d. h. > 80 Gew.-%, an CO enthalten.

Das in D₅ zugeführte CO-haltige Gase kann reines Kohlenmonoxid (>99 Gew.-% CO) sein oder aber auch ein Gas mit einem niedrigeren CO-Gehalt, mit zum Beispiel 90 bis 98 Gew.-%, vorzugsweise mit 95 bis 98 Gew.-% CO.

In Schritt D₅ liegt das Mischungsverhältnis der Flüssigphase (200b) und des CO-haltigen Gases (302a1), entspricht 200b/302a1, vorzugsweise im Bereich von 0,2 bis 0,6, vorzugsweise im Bereich von 0,3 bis 0,5. Typische Ströme im Betrieb sind 30,000 bis 48.000 kg/h für die Flüssigphase (200b), sowie 80,000 bis 150,000 kg/h für das CO-haltige Gas (302a1).

In Schritt D₉ erfolgt das Anreichern mit Kohlenmonoxid CO (400b) so, dass vorzugsweise ein CO-haltiges Gas (302a1) mit folgender Zusammensetzung erhalten wird: 0,1 bis 0,5 Gew.-%, vorzugsweise 0,2 bis 0,4 Gew.-% Wasserstoff H₂, 90 bis 99 Gew.-%, vorzugsweise 95 bis 98 Gew.-% Kohlenmonoxid CO, 1 bis 3 Gew.-%, vorzugsweise 1,5 bis 2,0 Gew.-% C8-Olefine, 0 bis 0,5 Gew.-%, vorzugsweise 0,2 bis 0,4 Gew.-% Nebenprodukte (z.B. Paraffine, Alkohole, etc.), und 0 bis 0,5 Gew.-%, vorzugsweise 0,2 bis 0,4 Gew.-% C9-Aldehyde.

In den erfindungsgemäßen Verfahren zur Herstellung von C9-Aldehyden oder von C9-Alkoholen kann eine Coldbox (400) vorgesehen sein. In diesem Fall kann in Schritt D₄) das in die Stripkolonne (200) zugeführte Kohlenmonoxid (400b) aus der Coldbox (400) kommen. Unabhängig von Schritt D₄) kann auch in Schritt D₉) das die Gasphase (302a) anreichernde Kohlenmonoxid (400b) aus der Coldbox (400) kommen. Gleiches gilt für Schritt D₁₀), bei welchem unabhängig von Schritten D₄) und D₉) der die Gasphase (302a2) anreichernde Wasserstoff H₂ (400a) aus der Coldbox (400) kommen kann. Es können auch mehrere Coldboxen (400) vorgesehen sein, wobei z.B. für jeden der Schritte D₄), D₉) und D₁₀) eine andere Coldbox (400) verwendet wird.

Die Coldbox oder die Coldboxen (400) werden dabei mit Synthesegas (400c) versorgt, welches von dem in den Reaktor (100) geführten Synthesegas (020a) abgezweigt werden kann. Selbstverständlich kann das Synthesegas (400c) auch aus einer anderen Synthesegasquelle zugeführt werden.

Gegenstand der vorliegenden Erfindung ist auch eine Anlage zur Herstellung von C9-Aldehyden. Optional kann die Anlage erweitert werden zur Herstellung von C9-Alkoholen. Die Anlage umfasst dabei folgende Elemente:
D₁₋₂) einen Reaktor (100) zum Hydroformylieren einer Reaktionsmischung unter Erhalt einer Hydroformylierungsmischung (100a), eine Zuführleitung in den Reaktor (100) für eine Zusammensetzung enthaltend mindestens 70 Gew.-% C8-Olefine (010) und eine Zuführleitung in den Reaktor (100) für Synthesegas (020b);
D₃) ein Entspannungsgefäß (101), eine Zuführleitung dorthin für die Hydroformylierungsmischung (100a), eine Abführleitung (101a) von dort für eine abgetrennte Gasphase und eine Abführleitung (101b) von dort für eine abgetrennte Flüssigphase, und eine Stripkolonne (200), in welche die Abführleitung (101b) leitet;
D₄) eine Zuführleitung für Kohlenmonoxid CO (400b) in die Stripkolonne (200), eine Abführleitung (200a) von dort für eine abgetrennte Gasphase und eine Abführleitung (200b) von dort für eine abgetrennte Flüssigphase, und einen Verdampfer (300), in welchen die Abführleitung (200b) leitet;
D₅) eine Zuführleitung (302a1) in den Verdampfer (300) für ein CO-haltiges Gases, eine Abführleitung (300a) von dort für eine abgetrennte Gasphase und eine Abführleitung (300b) von dort für eine abgetrennte Flüssigphase, einen Kondensator (301), in welchen die Abführleitung (300a) leitet, ein Entspannungsgefäß (302), eine Abführleitung (300c) für Kondensat vom Kondensator (301) in das Entspannungsgefäß (302);
D₆) eine Abführleitung (302a) von dem Entspannungsgefäß (302) für eine abgetrennte Gasphase und eine Abführleitung (300b) von dem Entspannungsgefäß (302) für eine abgetrennte Flüssigphase, und, **optional,** eine Destillationskolonne (500) zur Abtrennung von C9-Aldehyd, in welche die Abführleitung (302b) leitet, und eine Abführleitung (500a) für das abgetrennte C9-Aldehyd;
D₇) einen Kondensator (102) in den eine Zuführleitung (101a) für eine Gasphase leitet, eine Rückführleitung (101b) für ein Kondensat vom Kondensator (102) in das Entspannungsgefäß (101), eine Rückführleitung (102a) für ein abgetrenntes Recyclingsynthesegas vom Kondensator (102) in den Reaktor (100);
D₈) einen Kondensator (201) in den eine Zuführleitung (200a) für eine Gasphase leitet, eine Rückführleitung (201b) für ein Kondensat vom Kondensator (201) in die Stripkolonne (200), eine Rückführleitung (201a) für ein abgetrenntes Recyclingsynthesegas vom Kondensator (201) in den Reaktor (100);
D₉) eine Zuführleitung (400b) für Kohlenmonoxid CO zu der Abführleitung (302a) von dem Entspannungsgefäß (302), eine Verlängerungsleitung (302a1) für CO-haltiges Gas von der Abführleitung (302a) zu dem Verdampfer (300);
D₁₀) eine Abführleitung (302a2) abgehend von der Abführleitung (302a), eine Zuführleitung (400a) für Wasserstoff H₂ zu der Abführleitung (302a2), eine Rückführleitung (400a1) für ein gebildetes Recyclingsynthesgas von der Zuführleitung (400a) in den Reaktor (100);
E) **optional,** eine Teilanlage zum Hydrieren der über die Abführleitung (500a) abgeführten C9-Aldehyde zum Erhalt von C9-Alkoholen,
dadurch gekennzeichnet, dass die Anlage folgende weitere Elemente umfasst:
eine Purgestromleitung (102aP1) zum Entnehmen einer Gasmenge aus der Rückführleitung (102a), eine Purgestromleitung (201 aP2) zum Entnehmen einer Gasmenge aus der Rückführleitung (201a), und eine Purgestromleitung (302a2P3) zum Entnehmen einer Gasmenge aus der Abführleitung (302a2).

Die erfindungsgemäße Anlage zur Herstellung von C9-Aldehyden oder die erweiterte Anlage zur Herstellung von C9-Alkoholen kann um Teilanlagen zur Herstellung der benötigten Zusammensetzung enthaltend die C8-Olefine ergänzt werden. Ein solches ergänzte Anlage umfasst die den Elementen D₁) bis D₁₀) vorangestellten nachfolgenden Elemente:
A-B) eine Teilanlage zum Oligomerisieren eines Kohlenwasserstoffstroms, welcher n-Buten enthält, mithilfe eines Katalysatorsystems unter Erhalt eines C8-Olefine enthaltenden Oligomerisats; und
C) eine Teilanlage zum Abtrennen des Katalysatorsystems aus dem Oligomerisat und zum destillativen Aufarbeiten des Oligomerisats unter Erhalt eines Oligomerisierungsprodukts als Zusammensetzung (010).

Die erfindungsgemäßen Anlagen zur Herstellung von C9-Aldehyden oder C9-Alkoholen können eine Coldbox (400) umfassen. In diesem Fall kann in Element D₄) die Zuführleitung für Kohlenmonoxid CO (400b) aus der Coldbox (400) kommen. Unabhängig von Element D₄) kann auch in Element D₉) die Zuführleitung (400b) für Kohlenmonoxid CO aus der Coldbox (400) kommen. Gleiches gilt für Element D₁₀), bei welchem unabhängig von den Elementen D₄) und D₉) die Zuführleitung (400a) für Wasserstoff H₂ aus der Coldbox (400) kommen kann. Es können auch mehrere Coldboxen (400) vorgesehen sein, wobei z.B. in jedem der Elemente D₄), D₉) und D₁₀) eine andere Coldbox (400) verwendet wird.

Sind eine oder mehrere Coldboxen (400) vorhanden, führen dorthin eine oder mehrere Synthesegasleitungen (400c), die von der Zuführleitung für Synthesegas (020b) in den Reaktor (100) abzweigen. Selbstverständlich können die eine oder die mehreren Synthesegasleitungen (400c) auch von einer oder mehreren anderen Synthesegasquellen aus zu der oder den Coldboxen (400) geführt werden.

Im Rahmen der vorliegenden Erfindung ist bevorzugt, dass die C9-Aldehyde einer Zusammensetzung entsprechen, die Isononanal enthält, überwiegend enthält oder im Wesentlichen daraus besteht. Entsprechend ist bevorzugt, dass die C9-Alkohole einer Zusammensetzung entsprechen, die Isononanol enthält, überwiegend enthält oder im Wesentlichen daraus besteht. "Enthalten" bedeutet, dass die Zusammensetzung Isononanal bzw. Isononanol in einer Menge im Bereich von 10 Gew.-% bis 50 Gew.-% umfasst. "Überwiegend enthalten" bedeutet, dass die Zusammensetzung Isononanal bzw. Isononanol in einer Menge im Bereich von über 50 Gew.-% bis 90 Gew.-% umfasst. "Im Wesentlichen daraus bestehen" bedeutet, dass die Zusammensetzung Isononanal bzw. Isononanol in einer Menge im Bereich von über 90 Gew.-% bis 100 Gew.-% umfasst.

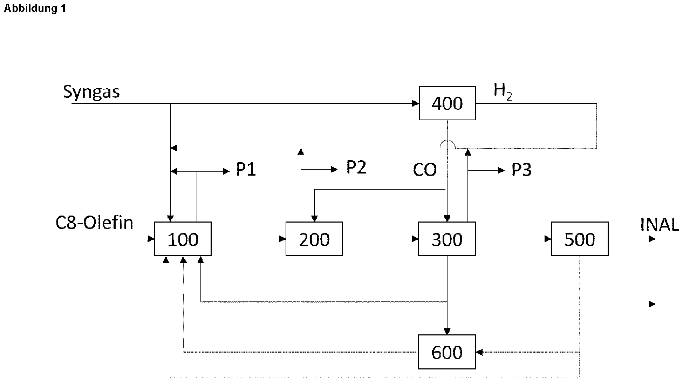

Vorteile, Einzelheiten und Merkmale der Erfindung werden anhand des im folgenden erläuterten Beispiels mit Verweis auf die Abbildungen 1, 2 und 3 deutlich.

Abbildung 1 zeigt die Unterteilung der erfindungsgemäßen Verfahrensschritte D) in 6 Abschnitte:
1. Hydroformylierung (Abschnitt 100),
2. Entfernung von gelöstem Wasserstoff H₂ (Abschnitt 200),
3. Abtrennung von Schwersiedern (Abschnitt 300),
4. Abtrennung von Kohlenmonoxid CO und Wasserstoff H₂ sowie Rückführung von Synthesegas (Abschnitt 400),
5. Aufreinigung INAL (Abschnitt 500), und
6. Rückgewinnung des Katalysators (Abschnitt 600).

Der Katalysator kann in die Reaktionszone (Abschnitt 100) zurückgeführt werden und zwar gelöst in Schwersiedern (aus Abschnitt 300) und/oder gelöst in nicht umgesetzten C8-Olefinen (aus Abschnitt 600). Der Synthesegasstrom wird in mindestens zwei Fraktionen aufgeteilt, wobei eine Fraktion mit wiederaufbereitetem Synthesegas gemischt und der Hydroformylierung (Abschnitt 100) zugeführt wird. Die andere Fraktion des Synthesegases wird in Abschnitt 400 geleitet. Dort wird, z.B. in einem Membrantrennverfahren oder in einer Coldbox, Kohlenmonoxid CO von Wasserstoff H₂ getrennt. Das CO wird in die Abschnitte 200 und 300 geleitet und dort verwendet. Die Gasströme aus den Abschnitten 200 und 300 werden mit dem H₂-Strom aus Abschnitt 400 gemischt und bilden so das wiederaufbereitete Synthesegas.

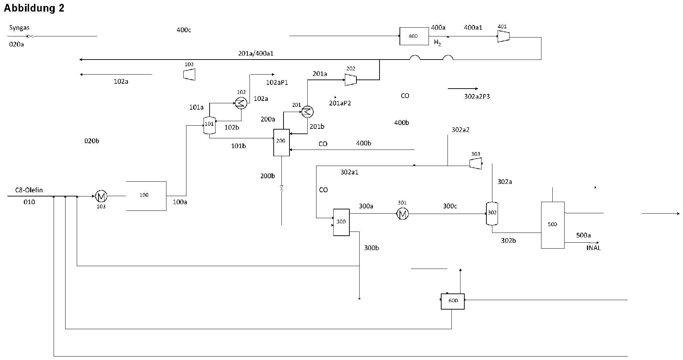

Abbildung 2 zeigt den Hydroformylierungsteil des erfindungsgemäßen Verfahrens detaillierter. Die Hydroformylierung zur Herstellung von Isononanal aus C8-Olefinen mit Synthesegas wurde simuliert. Die Simulation wurde mit der Software Aspen Plus durchgeführt, und der Aufbau des Eigenschaftsmodells wurde mit verfügbaren experimentellen Daten zu den thermophysikalischen Eigenschaften von reinen Komponenten und Gemischen validiert.

C8-Olefine mit einem Massendurchsatz von 23 t/h werden zusammen mit Synthesegas in den Hydroformylierungsreaktor (100) eingespeist. Das molare Verhältnis von CO zu H₂ im Synthesegas (020b) beträgt etwa 1 : 1 und CO wird mit einem stöchiometrischen Überschuss von 1,02 zugeführt. Die Reaktion wird bei 120 °C, 20 bar Druck und 100 ppm Rhodium-Katalysatorkonzentration durchgeführt. Die Konzentration der Komponenten im C8-Olefinspeisestrom beträgt: 8 Gew.-% n-Octene, 62 Gew.-% Methylheptene und 30 Gew.-% Dimethylhexene. In der Simulation wird die Umwandlung von C8-Olefinen und die INAL-Selektivität gemäß Tabelle 2 und Tabelle 3 berechnet. Es wird eine Reaktorverweilzeit von 180 Minuten verwendet. Die Dampfphase und die Flüssigphase des Reaktorabstroms werden im Entspannungsgefäß (101) bei dem Reaktorausgangsdruck und 90 °C getrennt. Ein Teil der nicht umgesetzten Olefine und Reaktionsprodukte im Brüdenstrom wird im Wärmetauscher (102) bei 45 °C kondensiert und in den Behälter (101) zurückgeführt.

Der Flüssigkeitsstrom aus (101) wird der H₂-Stripkolonne (200) zugeführt. Die Stripkolonne hat 2 theoretische Stufen und arbeitet mit einem Kopfdruck von 14,5 bar. Im Sumpf der Kolonne werden 130 Nm³/h CO zugeführt. Die H₂-Konzentration im Flüssigkeitsstrom wird von 0,72 mol% auf 0,12 mol% reduziert. Der Dampfstrom aus dem Kopf der Stripkolonne (200) wird auf 45 °C abgekühlt, und der kondensierte Flüssigkeitsstrom wird in die Kolonne c zurückgeführt.

Der Flüssigkeitsstrom aus dem Sumpf der Stripkolonne (200) wird im Gegenstrom mit 116.425 Nm³/h CO in den Fallfilmverdampfer (300) geleitet. Der Verdampfer arbeitet bei 120 °C und einem Druck von 1,5 bar. Im Flüssigkeitsstrom (Flüssigaustrag) des Verdampfers werden Hochsieder mit einer Konzentration von 5 Gew.-% INAL abgeschieden. Eine Fraktion der Hochsieder wird in den Reaktionsteil zurückgeführt und 564 kg/h werden dem Rh-Rückgewinnungsteil zugeführt. Das Massenverhältnis Einspeisung/Flüssigaustrag im Verdampfer wird in der Simulation auf 3,9 festgelegt. Der Dampfstrom aus dem Verdampfer (300) wird im Wärmetauscher (301) auf eine Temperatur von 20 °C abgekühlt und in den Entspannungsbehälter (302) geleitet, um die Gas- und Flüssigphase zu trennen. Die CO-haltige Gasphase wird zur Überwindung von Druckverlusten dem Verdichter (303) zugeführt. Ein Teil des Ausgangsstroms wird mit CO aus der Coldbox (400) gemischt und in den Verdampfer (300) zurückgeführt. Der Rest wird mit dem in der Coldbox (400) abgetrennten H₂-Strom gemischt. Das reformierte Synthesegas wird im mehrstufigen Kompressor mit Zwischenkühlung (401) auf einen Druck knapp über dem Druck des Reaktionsabschnitts zurückverdichtet.

Das INAL-Produkt wird in der Destillationskolonne (500) abgetrennt. Die Kolonne hat 33 theoretische Stufen, einen Teilkondensator, der bei 20 °C und einem Kopfdruck von 0,2 bar betrieben wird. Im Kopf der Kolonne werden nicht umgesetzte Olefine und Alkane abgetrennt. Im Sumpf der Kolonne werden 99,9 % INAL mit einer Reinheit von 99,84 Gew.-% gewonnen. Nicht umgesetzte C8-Olefine werden zum Teil in den Reaktor zurückgeführt, die andere Fraktion wird mit Ligand gemischt und dem Rh-Rückgewinnungsteil zugeführt. Der rezyklierte Strom an nicht umgesetzten C8-Olefinen beträgt in der Basissimulation 4,8 t/h und es werden 25,9 t/h INAL produziert.

Die Destillationskolonne in Abschnitt (500) weist einen Teilkondensator am Kopfende auf. C8-Aldehyde werden am Boden der Kolonne abgetrennt und die nicht umgesetzten C8-Olefine und Nebenprodukte (Paraffine, Alkohole und andere) am Kopf der Kolonne. Der flüssige Produktstrom am Kopf der Destillationskolonne wird in drei Teile aufgeteilt: (1) Eine Fraktion wird in den Abschnitt (600) geleitet und im Prozess zur Rückgewinnung von Rhodium verwendet, (2) die andere Fraktion wird in den Reaktionsabschnitt (100) zur weiteren Reaktion der nicht umgesetzten C8-Olefine geleitet, um auf diese Weise den erforderlichen Einsatzfaktor (t INA/t C8-Olefin = x) zu erreichen, und (3) die dritte Fraktion wird gespült, um eine Anhäufung der Nebenprodukte (Paraffine und andere) zu vermeiden.

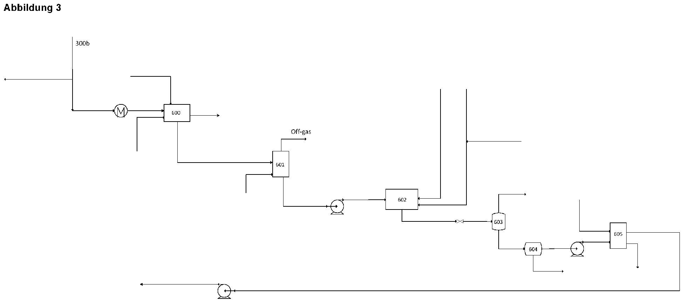

Abbildung 3 zeigt beispielhaft die Rückgewinnung des Katalysators (Abschnitt 600). Hochsieder mit einer Konzentration von 389 ppm Rhodium werden dem Abschnitt 600 zugeführt. Die Oxidation wird bei 65 °C mit Luft und 50-70 Gew.-% wässriger Essigsäure vorgenommen. Die Oxidationsreaktion kann in einem oder mehreren in Reihe geschalteten Reaktoren erfolgen. Jedem Reaktor ist ein Dekanter zur Trennung der organischen und der wässrigen Phase nachgeschaltet. Die organische Produktphase aus dem ersten Reaktor wird in den nächsten Reaktor geleitet und mit Luft und Essigsäure behandelt. Alle wässrigen Produktströme der Oxidationsreaktion werden gemischt und mit Stickstoff N₂ in das Entspannungsgefäß (601) geleitet. Das flüssige Produkt wird dann in den Reaktor (602) zur Extraktion von Rhodium in Synthesegas-Atmosphäre zum Gemisch aus nicht umgesetzten C8-Olefinen und Ligand geleitet. Der Reaktor arbeitet bei einer Temperatur im Bereich von 65-100 °C und einem Druck zwischen 3,5-20 bar. Die Gasphase wird im Entspannungsgefäß (603) und die wässrige Phase im Dekanter (604) abgetrennt. Um Spuren von Säure zu entfernen, wird die organische Phase aus dem Dekanter (604), die den Liganden-Rh-Komplex enthält, in der Extraktionssäule (605) mit einem wasserlöslichen Amin gewaschen, z. B. Triethanolamin. Das in der organischen Phase zurückgewonnene Rhodium wird in den Hydroformylierungsreaktor (100) zurückgeführt.

Bei der Hydroformylierung von C8-Olefinen wird Synthesegas (020, 102a, 201a, 400a1) als Reaktant verwendet. Aus dem Synthesegas abgetrenntes CO (400b) wird in der Stripkolonne (200) und/oder im Fallfilmverdampfer (300) zur Entfernung von H₂ verwendet. Eine Fraktion (302a2) des CO-reichen Gasstroms aus dem Abschnitt (300) wird mit H₂ gemischt, um dein neu gebildete Synthesegasgas (400a1) zu bilden, welches in den Hydroformylierungsreaktor (100) zurückgeführt wird. Auch überschüssiges Synthesegasgas, das im Behälter (101) abgetrennt wird, wird in den Reaktor (100) zurückgeführt.

Für den Fall, dass das molare Verhältnis von H₂ zu CO im frischen Synthesegasstrom (020a) 1 : 1 beträgt, werden die Entnahmen von CO/H₂-haltigen Gasen über die Purgeströme (102aP1), (201aP2) und (302a2P3) so eingestellt, dass die Recyclinggasströme (102a), (201a) und (400a1) in Summe ein Molverhältnis H₂ zu CO im Bereich von 1 : 1 aufweisen.

Für den Fall, dass das molare Verhältnis von H₂ zu CO im frischen Synthesegasstrom (020a) nicht 1 : 1 beträgt, werden die Entnahmen von CO/H₂-haltigen Gasen über die Purgeströme (102aP1), (201aP2) und (302a2P3) so eingestellt, dass der Synthesegasstrom (020b) einen Wert aufweist, der im Bereich von 1 : 1 liegt.

Der Einsatz von frischem Synthesegas (020a) kann durch die Rückgewinnung überschüssigen Synthesegases im Abschnitt 100 (102a) und Wiederverwendung in der Hydroformylierung (100) bzw. durch Neubildung und Wiederverwendung der Synthesegase in den Abschnitten 200 (201a) und 400 (400a1) minimiert werden.

## Patentansprüche

1. Verfahren zur Herstellung von C9-Aldehyden, wobei das Verfahren nachfolgende Schritte umfasst:
A) Bereitstellen eines Kohlenwasserstoffstroms, der n-Butene enthält;
B) Oligomerisieren des Kohlenwasserstoffstroms mithilfe eines homogenen Katalysatorsystems, welches Nickel und ein Aluminiumalkyl umfasst, unter Erhalt eines Oligomerisats, welches C8-Olefine enthält;
C) Abtrennen des Katalysatorsystems aus dem Oligomerisat und destillative Aufarbeitung des vom Katalysatorsystem befreiten Oligomerisats, wodurch ein Oligomerisierungsprodukt als Zusammensetzung (010) erhalten wird, welches mindestens 70 Gew.-% C8-Olefine enthält;
D₁) Zuführen einer Zusammensetzung (010) aus Schritt C) in einen Reaktor (100) und Zuführen einer Mischung von Wasserstoff H₂ und Kohlenmonoxid CO, also Synthesegas (020b), in denselben Reaktor (100) unter Erhalt einer Reaktionsmischung;
D₂) Hydroformylieren der Reaktionsmischung in dem Reaktor (100) mithilfe eines Katalysatorsystem, welches Cobalt oder Rhodium und einen Liganden umfasst, unter Erhalt einer Hydroformylierungsmischung (100a), welche zumindest C9-Aldehyde enthält;
D₃) Zuführen der Hydroformylierungsmischung (100a) in ein Entspannungsgefäß (101), dortiges Abtrennen einer Gasphase (101a) und einer Flüssigphase (101b) aus der Hydroformylierungsmischung (100a) und Überführen der Flüssigphase (101b) in eine Stripkolonne (200);
D₄) Zuführen von Kohlenmonoxid CO (400b) in die Stripkolonne (200), dortiges Abtrennen einer Gasphase (200a) und einer Flüssigphase (200b) aus der Flüssigphase (101b) und Überführen der Flüssigphase (200b) in einen Verdampfer (300);
D₅) Zuführen eines CO-haltigen Gases (302a1) in den Verdampfer (300), dortiges Abtrennen einer Gasphase (300a) und einer Flüssigphase (300b) und Überführen der Gasphase (300a) in einen Kondensator (301) und Überführen des Kondensats (300c) in ein Entspannungsgefäß (302);
D₆) in dem Entspannungsgefäß (302) Abtrennen einer Gasphase (302a) und einer C9-Aldehyd-haltigen Flüssigphase (302b) aus der Flüssigphase (300c);
D₇) Zuführen der Gasphase (101a) aus Schritt D₃ in einen Kondensator (102), in dem Kondensator (102) Abtrennen eines Kondensats (102b) und Rückführen des Kondensats (102b) in das Entspannungsgefäß (101), und in dem Kondensator (102) Abtrennen eines Recyclingsynthesegases (102a) und Rückführen des Recyclingsynthesegases (102a) in den Reaktor (100);
D₈) Zuführen der Gasphase (200a) aus Schritt D₄ in einen Kondensator (201), in dem Kondensator (201) Abtrennen eines Kondensats (201b) und Rückführen des Kondensats (201b) in die Stripkolonne (200), und in dem Kondensator (201) Abtrennen eines Recyclingsynthesegases (201a) und Rückführen des Recyclingsynthesegases (201a) in den Reaktor (100);
D₉) Anreichern einer Teils der Gasphase (302a) aus Schritt D₆ mit Kohlenmonoxid CO (400b) und Rückführen des so erhaltenen CO-haltigen Gases (302a1) in den Verdampfer (300);
D₁₀) Anreichern eines verbleibenden Teils (302a2) der Gasphase (302a) mit Wasserstoff H₂ (400a) unter Bildung eines Recyclingsynthesgas (400a1) und Rückführen desselben in den Reaktor (100);
**dadurch gekennzeichnet, dass** von dem Recyclingsynthesgas (102a) eine solche Gasmenge als Purgestrom (102aP1), von dem Recyclingsynthesgas (201a) eine solche Gasmenge als Purgestrom (201aP2) und/oder von dem CO-haltigen Gas (302a2) eine solche Gasmenge als Purgestrom (302aP3) entnommen und verworfen wird, dass die Summe der Recyclinggasströme (102a), (201a) und (400a1) ein Molverhältnis H₂ zu CO im Bereich von 0,9 bis 1,1 aufweist, oder dass die Recyclinggasströme (102a), (201a) und (400a1) mit frischem Synthesegas (020a) gemischt werden und das dadurch gebildete Synthesegas (020b) ein Molverhältnis H₂ zu CO im Bereich von 0,9 bis 1,1 aufweist.

2. Verfahren nach Anspruch 1, wobei die Aluminiumverbindung des Katalysatorsystems in Schritt B) Metyhlaluminiumdichlorid (MeAlCl₂), Ethylaluminiumdichlorid (EtAlCl₂), Diethylaluminiumchlorid (Et₂AlCl), Diisobutylaluminiumchlorid (iBu₂AlCl) und Isobutylaluminiumdichlorid (iBuAlCl₂). Eine besonders bevorzugte Aluminiumverbindung ist Ethylaluminiumdichlorid (EtAlCl₂) oder eine Mischung davon ist.

3. Verfahren nach Anspruch 1 oder 2, wobei die Nickelverbindung des Katalysatorsystems in Schritt B) Nickel(II)-chlorid, Nickel(II)(dimethoxyethan)-chlorid, Nickel(II)-bromid, Nickel(II)(dimethoxyethan)-bromid, Nickel(II)-fluorid, Nickel(II)-iodid, Nickel(II)-sulfat, Nickel(II)-carbonat, Nickel(II)-dimethylglyoxim, Nickel(II)-hydroxid, Nickel(II)-hydroxyacetat, Nickel(II)-oxalat, Nickel(II)-carboxylate wie zum Beispiel Nickel-2-ethylhexanoat, Nickel(II)-phenate, Nickel(II)-naphthenate, Nickel(II)-acetat, Nickel(II)-trifluoracetat, Nickel(II)-triflat, Nickel(II)-acetylacetonat, Nickel(II)-hexafluoroacetylacetonat, π-Allylnickel(II)-chlorid, π-Allylnickel(II)-bromid, Methallylnickel(II)-chlorid-Dimer, η3-Allylnickel(II)-hexafluorophosphat, η3-Methallylnickel(II)-hexafluorophosphat, Nickel(II)-1,5-Cyclooctadienyl oder eine Mischung davon ist.

4. Verfahren nach einem der vorhergehenden Ansprüche, wobei die Oligomerisierung in Schritt B) bei einer Temperatur von -20° C. bis 80° C durchgeführt wird.

5. Verfahren nach einem der vorhergehenden Ansprüche, wobei die Oligomerisierung in einer Reaktionszone durchgeführt wird, die mehrere identische oder unterschiedliche Reaktoren enthält, vorzugsweise eine Kaskade aus mehreren hintereinander geschalteten Reaktoren vorliegt.

6. Verfahren nach einem der vorhergehenden Ansprüche, wobei eine Coldbox (400) vorgesehen ist und in Schritt D₄) das in die Stripkolonne (200) zugeführte Kohlenmonoxid (400b) aus der Coldbox (400) kommt, und/oder wobei in Schritt D₉) das die Gasphase (302a) anreichernde Kohlenmonoxid (400b) aus der Coldbox (400) kommt, und/oder wobei in Schritt D₁₀) der die Gasphase (302a2) anreichernde Wasserstoff H₂ (400a) aus der Coldbox (400) kommt.

7. Verfahren nach einem der vorhergehenden Ansprüche, wobei die Coldbox (400) mit Synthesegas (400c) versorgt wird, welches von dem Synthesegas (020a) abgezweigt wird.

8. Verfahren nach einem der vorhergehenden Ansprüche, wobei die in Schritt D₆) abgetrennte C9-Aldehyd-haltige Flüssigphase (302b) zur Abtrennung der C9-Aldehyde (500a) in eine Destillationskolonne (500) überführt wird.

9. Verfahren nach einem der vorhergehenden Ansprüche, wobei sich an den Schritte D₁) bis D₁₀) ein Schritt E) anschließt, in welchem die erhaltenen C9-Aldehyde (500a) unter Erhalt von C9-Alkoholen hydriert werden.

10. Anlage zur Herstellung von C9-Aldehyden, **optional** zur Herstellung von C9-Alkoholen, wobei die Anlage folgende Elemente umfasst:
A-B) eine Teilanlage zum Oligomerisieren eines n-Buten-haltigen Kohlenwasserstoffstroms mithilfe eines Katalysatorsystems unter Erhalt eines Oligomerisats, das C8-Olefine enthält;
C) eine Teilanlage zum Abtrennen des Katalysatorsystems aus dem Oligomerisat und geeignet zur destillativen Aufarbeitung des Oligomerisats unter Erhalt eines Oligomerisierungsprodukts (010);
D₁₋₂) einen Reaktor (100) zum Hydroformylieren einer Reaktionsmischung unter Erhalt einer Hydroformylierungsmischung (100a), eine Zuführleitung in den Reaktor (100) für das Oligomerisierungsprodukt (010) und eine Zuführleitung in den Reaktor (100) für Synthesegas (020b);
D₃) ein Entspannungsgefäß (101), eine Zuführleitung dorthin für die Hydroformylierungsmischung (100a), eine Abführleitung (101a) von dort für eine abgetrennte Gasphase und eine Abführleitung (101b) von dort für eine abgetrennte Flüssigphase, und eine Stripkolonne (200), in welche die Abführleitung (101b) leitet;
D₄) eine Zuführleitung für Kohlenmonoxid CO (400b) in die Stripkolonne (200), eine Abführleitung (200a) von dort für eine abgetrennte Gasphase und eine Abführleitung (200b) von dort für eine abgetrennte Flüssigphase, und einen Verdampfer (300), in welchen die Abführleitung (200b) leitet;
D₅) eine Zuführleitung (302a1) in den Verdampfer (300) für ein CO-haltiges Gases, eine Abführleitung (300a) von dort für eine abgetrennte Gasphase und eine Abführleitung (300b) von dort für eine abgetrennte Flüssigphase, einen Kondensator (301), in welchen die Abführleitung (300a) leitet, ein Entspannungsgefäß (302), eine Abführleitung (300c) für Kondensat vom Kondensator (301) in das Entspannungsgefäß (302);
D₆) eine Abführleitung (302a) von dem Entspannungsgefäß (302) für eine abgetrennte Gasphase und eine Abführleitung (300b) von dem Entspannungsgefäß (302) für eine abgetrennte Flüssigphase, und, **optional,** eine Destillationskolonne (500) zur Abtrennung von C9-Aldehyd, in welche die Abführleitung (302b) leitet, und eine Abführleitung (500a) für das abgetrennte C9-Aldehyd;
D₇) einen Kondensator (102) in den eine Zuführleitung (101a) für eine Gasphase leitet, eine Rückführleitung (101b) für ein Kondensat vom Kondensator (102) in das Entspannungsgefäß (101), eine Rückführleitung (102a) für ein abgetrenntes Recyclingsynthesegas vom Kondensator (102) in den Reaktor (100);
D₈) einen Kondensator (201) in den eine Zuführleitung (200a) für eine Gasphase leitet, eine Rückführleitung (201b) für ein Kondensat vom Kondensator (201) in die Stripkolonne (200), eine Rückführleitung (201a) für ein abgetrenntes Recyclingsynthesegas vom Kondensator (201) in den Reaktor (100);
D₉) eine Zuführleitung (400b) für Kohlenmonoxid CO zu der Abführleitung (302a) von dem Entspannungsgefäß (302), eine Verlängerungsleitung (302a1) für CO-haltiges Gas von der Abführleitung (302a) zu dem Verdampfer (300);
D₁₀) eine Abführleitung (302a2) abgehend von der Abführleitung (302a), eine Zuführleitung (400a) für Wasserstoff H₂ zu der Abführleitung (302a2), eine Rückführleitung (400a1) für ein gebildetes Recyclingsynthesgas von der Zuführleitung (400a) in den Reaktor (100);
E) **optional,** eine Teilanlage zum Hydrieren der über die Abführleitung (500a) abgeführten C9-Aldehyde zum Erhalt von C9-Alkoholen,
**dadurch gekennzeichnet, dass** die Anlage folgende weitere Elemente umfasst:
eine Purgestromleitung (102aP1) zum Entnehmen einer Gasmenge aus der Rückführleitung (102a),
eine Purgestromleitung (201aP2) zum Entnehmen einer Gasmenge aus der Rückführleitung (201a), und
eine Purgestromleitung (302a2P3) zum Entnehmen einer Gasmenge aus der Abführleitung (302a2).

11. Anlage nach Anspruch 10, wobei die Anlage eine Coldbox (400) umfasst, und in Element D₄) die Zuführleitung für Kohlenmonoxid CO (400b) aus der Coldbox (400) kommt, und/oder wobei in Element D₉) die Zuführleitung (400b) für Kohlenmonoxid CO aus der Coldbox (400) kommt, und/oder wobei in Element D₁₀) die Zuführleitung (400a) für Wasserstoff H₂ aus der Coldbox (400) kommt.

12. Anlage nach Anspruch 11, wobei eine Synthesegasleitung (400c) von der Zuführleitung für Synthesegas (020b) abzweigt und zu der Coldbox führt (400).

13. Verfahren oder Anlage nach einem der vorhergehenden Ansprüche, wobei die C9-Aldehyde einer Zusammensetzung entsprechen, die Isononanal enthält, überwiegend enthält oder daraus besteht, und/oder wobei die C9-Alkohole einer Zusammensetzung entsprechen, die Isononanol enthält, überwiegend enthält oder daraus besteht.
